# FASCICULE DE BREVET EUROPEEN

(11) **EP 1 789 137 B1**
(45) Date de publication et mention de la délivrance du brevet: **04.09.2013**
(21) Numéro de dépôt: 05791120.8
(22) Date de dépôt: 19.07.2005
(51) Int. Cl.: A61N 7/02, A61N 7/00, A61B 17/22

(54) **DISPOSITIF DE TRAITEMENT PAR ULTRASONS**
ULTRASCHALLBEHANDLUNGSVORRICHTUNG
ULTRASOUND TREATING DEVICE

(30) Priorité: 23.07.2004 FR 0408180; 13.08.2004 FR 0408874
(43) Date de publication de la demande: 30.05.2007
(73) Titulaire: INSERM, 75654 Paris Cédex 13 (FR); Theraclion, 75015 Paris (FR)
(72) Inventeur: LACOSTE, François, F-75014 Paris (FR); CHAPELON, Jean-Yves, F-69100 Villeurbanne (FR); PICHOT, Olivier, F-38700 Corenc (FR); MILLERET, René, F-34000 Montpellier (FR); PICHARDO, Samuel, 2075 Bayview Ave. Rm C713, Toronto ON M4N 3M5 (CA)
(74) Mandataire: Hirsch & Associés
(86) Numéro de dépôt international: PCT/FR2005/001842
(87) Numéro de publication internationale: WO 2006/021651

(56) Documents cités:
- WO-A-01/02055
- WO-A-01/17455
- WO-A-01/34018
- GB-A- 2 113 099
- US-A- 4 459 852
- US-B1- 6 206 843
- US-B1- 6 374 132

## Description

### DOMAINE TECHNIQUE

La présente invention concerne un dispositif de traitement par ultrasons, en particulier pour le traitement de veines variqueuses.

Le système veineux comprend un réseau profond intramusculaire et un réseau superficiel sous cutané qui assurent parallèlement le retour du sang veineux. Dans ces deux réseaux, l'hémodynamique veineuse est dépendant de différents mécanismes qui génèrent un gradient de pression permettant de propulser et d'aspirer le sang veineux vers le coeur droit. Au niveau des membres inférieurs, malgré un gradient de pression faible et variable selon les conditions physiologiques, le retour veineux reste efficace en toute circonstance grâce à l'existence de valvules disposées dans toutes les veines des membres inférieurs, qui constituent de véritables dispositifs anti-reflux.

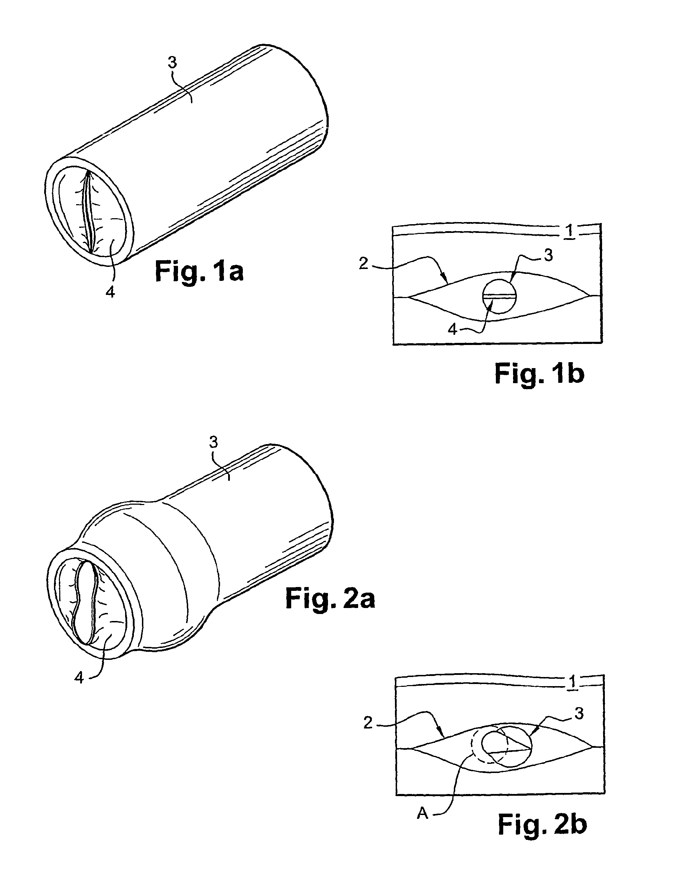

Les figures 1a et 1b illustrent une veine saine. La veine 3 est illustrée avec une valvule 4 saine avec des feuillets se refermant bord à bord pour assurer la fonction d'anti-reflux sanguin. La veine 3 du réseau superficiel est située dans un fascia 2 sous la peau 1.

Les varices, qui caractérisent la maladie veineuse superficielle, concernent le réseau veineux superficiel des membres inférieurs. Cette pathologie se définit sur le plan morphologique par une augmentation anormale du calibre de la veine et sur le plan hémodynamique par l'existence d'un reflux lié à un dysfonctionnement des valvules veineuses.

Les figures 2a et 2b illustrent une veine variqueuse. La veine 3 est illustrée avec une valvule 4 pathologique avec des feuillets ne se refermant pas complètement. La fonction d'anti-reflux sanguin n'est donc plus assurée parfaitement. Une portion déhiscente A apparaît alors sur la paroi de la veine 3 à l'intérieur du fascia 2.

Si les connaissances sur l'origine et la nature de la maladie variqueuse restent encore limitées, l'hypothèse d'une atteinte primitive de la paroi de la veine est actuellement admise. L'exploration du réseau veineux superficiel des membres inférieurs par écho Doppler a beaucoup fait progresser les connaissances dans ce domaine. On peut grâce à cette technique étudier le calibre de la veine malade et mesurer le reflux, mais aussi identifier les valvules pathologiques et étudier précisément les caractéristiques du reflux de la veine variqueuse.

### ETAT DE LA TECHNIQUE

Les traitements thérapeutiques classiques reposent sur l'exérèse chirurgicale des veines pathologiques.

Depuis peu un traitement conservateur a été essayé pour un stade de début de l'insuffisance veineuse superficielle. Le plus souvent, en effet, on peut observer au stade initial de la maladie variqueuse une dilatation de la paroi veineuse au niveau des anneaux d'insertion de la valvule préterminale (située en haut de la cuisse) et de la valvule du canal fémoral (en bas de la cuisse). Restaurer la compétence de ces valvules permet, dans 90 % des cas où la mise en oeuvre de ce traitement est possible, de limiter l'évolution ultérieure vers l'insuffisance veineuse variqueuse. La seule technique thérapeutique pratiquée dans ce but actuellement disponible est chirurgicale. Il s'agit de la plastie par manchonnage, qui consiste à entourer le segment veineux dilaté par un anneau de Téflon ou de silicone pour en réduire le calibre jusqu'à restaurer le bon fonctionnement de la valvule. Un tel traitement a été décrit dans la publication « The treatment of varicose veins with external stenting to the Saphenofemoral junction » de R.J.Lane, M.L.Cuzzilla, et J.C.Coroneos, Vasc. Endovasc. Surg. 3 6, 2002, pp 179-192.

Un autre traitement connu est la sclérothérapie, qui consiste en l'injection dans la veine de produit sclérosant. Cette technique de traitement est cependant difficilement maîtrisable.

D'autres traitements utilisent la chaleur, soit pour coaguler le sang situé dans la veine et ainsi obtenir une thrombose, soit pour réduire ou obturer le diamètre de la veine. Ces traitements provoquent d'abord l'oblitération de la portion de veine variqueuse, puis sa fibrose et sa lyse par des macrophages. Les échographies de contrôle à 18 mois montrent une disparition totale de la paroi veineuse pathologique. Par exemple le système décrit dans le brevet US-A-6 613 045 dit "fermeture", de l'anglais "closure", mis au point par la société VNUS, utilise des radiofréquences pour chauffer la portion de veine variqueuse. Un autre système décrit dans le brevet US-A-6 398 777 utilise de la chaleur apportée par un laser.

D'autres traitements ont été proposés qui utilisent de la chaleur apportée par dès ultrasons.

Par exemple, la publication de Delon-Martin et al « Venous thrombosis generation by means of high intensity focused ultrasound », Ultrasound in Med and Biol, 1995, 21 (1), p 113-119, décrit des essais avec des ultrasons de puissance. Les ultrasons sont cependant focalisés dans la veine, le but étant de coaguler le sang et non pas le tissu veineux ou périphérique.

Par ailleurs, la publication de Schultz-Haakh et al « Ultrasonic Treatment of Varicose Veins », Angiology. Février 1989, 40 (2), p 129-137 décrit une destruction de la paroi veineuse avec des ultrasons focalisés. Des tirs juxtaposés d'ultrasons sont appliqués afin de détruire la paroi pour provoquer l'occlusion de la veine.

Le brevet US-A-6 436 061 décrit une méthode consistant à chauffer le tissu situé entre la veine et la peau. Ce document propose aussi de détériorer l'endothélium de la veine pour provoquer des thromboses secondaires. Bien que les auteurs reconnaissent que la maladie ait pour origine le dysfonctionnement des valvules, ils n'agissent pas sur celles-ci ni sur la veine elle-même, mais au contraire ils cherchent à oblitérer complètement la veine.

Le brevet US-A-6 083 159 décrit une méthode de coagulation du sang par l'application d'ultrasons focalisés. La méthode décrite dans ce document ne prévoit pas non plus de traiter les valvules.

Le brevet US-A-6 676 601 propose de viser une cible à coaguler grâce à un transducteur intégré fonctionnant en mode Doppler.

Le brevet US-A-5 230 334 décrit l'application d'ultrasons focalisés de haute fréquence (>20MHz) pour contracter le collagène présent dans la cornée. Il est recommandé de ne pas chauffer à plus de 60-70°C et de limiter l'exposition thermique à quelques secondes. Cependant, aucunes combinaisons correctes de paramètres acoustiques ne sont données (fréquence, puissance, intensités, temps d'exposition) pour permettre de rétracter effectivement la cornée sans destruction collatérale.

Par ailleurs, les dispositifs à ultrasons focalisés de forte intensité, connus sous le terme de HIFU (pour « High Intensity Focused Ultrasound » en anglais) comportent généralement un transducteur de thérapie émettant une surface d'onde de forme sphérique, de sorte que l'énergie acoustique est concentrée en une tache ponctuelle. Si l'on veut traiter une ligne ou un volume de tissu, il faut déplacer le point focal grâce à un mouvement mécanique du transducteur ou à un déplacement électronique du point focal.

Les brevets US-A-6 656 136 et US-A-5 762 066 prévoient l'utilisation de transducteurs émettant des surfaces d'onde asphériques, de manière à élargir le point focal. Ces documents ne prévoient cependant pas d'utiliser des surfaces permettant de produire une ligne très fine, de longueur donnée.

Le brevet US-A-4 938 216 propose d'utiliser un transducteur émettant une surface d'onde de forme cylindrique de manière à produire une tache focale allongée sensiblement égale à la longueur du transducteur. La concentration acoustique d'un tel transducteur est cependant limitée.

### RESUME DE L'INVENTION

Il existe donc un besoin pour un dispositif de traitement par ultrasons qui permette de traiter une cible présentant la forme d'un segment de droite, telle qu'une paroi de veine variqueuse par exemple.

A cet effet, l'invention propose un dispositif à ultrasons comprenant un premier transducteur de traitement adapté à émettre un faisceau d'ondes ultrasonores vers une zone à traiter, le faisceau présentant une surface d'onde asphérique et créant une tache focale sur la zone à traiter allongée et inclinée par rapport à la direction de propagation des ondes ultrasonores, la longueur de la tache focale étant inférieure à la longueur du transducteur et la surface d'onde asphérique n'étant pas cylindrique.

Selon une caractéristique, la tache focale est perpendiculaire à la direction de propagation des ondes ultrasonores.

Selon une caractéristique, le dispositif comprend en outre un deuxième transducteur d'imagerie adapté à visualiser la zone à traiter.

Le document WO-A-01 34018 décrit un dispositif à ultrasons permettant de localiser une plaie perforante dans une artère et de la refermer par application d'ondes ultrasonores de haute intensité (HIFU).

Le document GB-A-2 113 099 décrit un appareil pour visualiser et traiter des tissus du corps humain par la chaleur en utilisant des ultrasons.

Le document US-A-6 206 843 décrit un système à ultrasons pour produire une pression d'onde acoustique sur un organe.

Le document WO-A-01 02055 décrit un appareil pour le traitement des tissus sous-cutanés par focalisation de vibrations ultrasonores.

Le document WO-A-01 17455 décrit une technique d'occlusion de structures anatomiques tubulaires telles que des vaisseaux sanguins par application d'énergie ultrasonore.

Selon les modes de réalisation, le transducteur d'imagerie est adapté à visualiser un plan perpendiculaire à un grand axe de la tache focale ou un plan incliné par rapport au grand axe de la tache focale.

Selon les modes de réalisation, le transducteur de traitement émet une surface d'onde de forme conique ou de forme ellipsoïdale ; ou de forme sphérique associée à une lentille cylindrique ou de forme plane associée à une lentille elliptique.

Selon un autre mode de réalisation, le transducteur de traitement émet une surface d'onde définie comme l'enveloppe d'une pluralité de cercles respectivement centrés sur une pluralité de points de la ligne focale et connectés entre eux de manière à ce que les deux normales en chaque points de l'enveloppe coupent la ligne focale.

Selon un mode de réalisation, le transducteur de traitement est découpé une pluralité de parties indépendantes adaptées à émettre chacune des ondes ultrasonores à la même fréquence et déphasées entre elles de manière à former une surface d'onde asphérique.

Selon un mode de réalisation, le dispositif comprend une pluralité de transducteurs de traitement adaptés à focaliser des ondes ultrasonores en une même tache focale sur une zone à traiter.

Selon un mode de réalisation, le transducteur d'imagerie est solidaire du transducteur de traitement.

Selon une caractéristique, le dispositif comprend en outre des moyens de contrôle de la fréquence et/ou de la puissance des ondes ultrasonores émises par le premier transducteur de traitement et/ou de la durée d'émission des ondes ultrasonores sur la surface à traiter.

Selon une caractéristique, les moyens de contrôle sont adaptés à interpréter des données fournies par le transducteur d'imagerie.

L'invention concerne ainsi un dispositif à ultrasons comprenant un premier transducteur de traitement adapté à émettre un faisceau d'ondes ultrasonores vers une zone à traiter, le faisceau présentant une surface d'onde asphérique, autre que cylindrique, et créant une tache focale sur la zone à traiter allongée et inclinée par rapport à la direction de propagation des ondes ultrasonores.

### BREVE DESCRIPTION DES FIGURES

D'autres caractéristiques et avantages de l'invention apparaîtront à la lecture de la description détaillée qui suit des modes de réalisation de l'invention, donnés à titre d'exemple uniquement et en références aux dessins qui montrent :
- figures 1a et 1b, des vues schématiques, respectivement en perspective et en coupe, d'une veine saine ;
- figures 2a et 2b, des vues schématiques, respectivement en perspective et en coupe, d'une veine variqueuse ;
- figure 3, une vue schématique d'un dispositif de traitement par ultrasons selon l'invention ;
- figures 4a à 4c, des vues schématiques, respectivement parallèle, oblique et perpendiculaire à la veine, d'un transducteur ultrasons selon un premier mode de réalisation du dispositif selon l'invention ;
- figures 5a à 5c, des vues schématiques, respectivement parallèle, oblique et perpendiculaire à la veine, d'un transducteur ultrasons selon un deuxième mode de réalisation du dispositif selon l'invention ;
- figure 6, une vue schématique, de transducteurs ultrasons selon un troisième mode de réalisation du dispositif selon l'invention ;
- figure 7, une vue schématique, de transducteurs ultrasons selon un quatrième mode de réalisation du dispositif selon l'invention.
- Figure 8, une représentation schématique d'une courbe servant de base à la construction de la surface d'onde d'un transducteur selon un cinquième mode de réalisation.

### EXPOSE DETAILLE DES MODES DE REALISATION DE L'INVENTION

Le dispositif de traitement par ultrasons selon l'invention comprend un premier transducteur de traitement adapté à émettre un faisceau d'ondes ultrasonores, par exemple des ondes ultrasonores de haute intensité (HIFU), vers une zone à traiter. La zone à traiter peut être par exemple une veine variqueuse.

Le transducteur de traitement émet un faisceau d'ondes présentant une surface d'onde asphérique et créant une tache focale sur la zone à traiter allongée et inclinée par rapport à la direction de propagation des ondes ultrasonores.

On définit la tache focale, de manière connue en soi, comme une zone de concentration des ondes ultrasonores sur une surface avec une concentration supérieure à un seuil donné.

La direction de propagation peut être définie comme la somme vectorielle des directions des rayons du faisceau d'ondes, pondérée par l'intensité. Ainsi, dans le cas de rayons iso intensités, la direction de propagation correspond à la moyenne des directions des rayons du faisceau d'ondes.

La surface d'onde est définie comme la surface équiphase de l'onde émise. La surface d'onde émise par le transducteur de traitement est telle que sa tache focale est allongée. On définit une tache allongée comme une tache présentant une dimension supérieure aux autres. Ainsi, la largeur de la tache focale est essentiellement limitée par la diffraction alors que la longueur de la tache correspond à plusieurs fois sa largeur de diffraction, au moins trois ou quatre fois. La profondeur de la tache focale est limitée par la pénétration des ondes de façon connue en soi.

La longueur de la tache focale est inférieure à la longueur du transducteur. On a ainsi une concentration de la puissance ultrasonore focalisée améliorée par rapport à une ligne focale obtenue avec un transducteur cylindrique classique. Le transducteur selon l'invention présente une surface la plus large et la plus longue possible afin de concentrer au maximum le faisceau d'ondes ultrasonores émis.

Le dispositif selon l'invention est donc particulièrement adapté au traitement de zone allongée, telle que des portions de veines englobant le point d'attache d'une valvule pathologique.

Dans la description qui suit, on utilise les mots de longueur, largueur, épaisseur, en référence à la veine constituant la zone à traiter représentée sur les figures. Ces références de positionnement sont illustratives et ne doivent pas être comprises comme limitant la position du dispositif en fonctionnement.

De même, la description qui suit est faite en référence à une zone à traiter constituée par une portion de veine variqueuse, mais il est entendu qu'il ne s'agit que d'un exemple illustratif et que le dispositif et la méthode selon l'invention sont applicables à toute autre zone à traiter, tel que par exemple une tumeur solide, par exemple dans le sein, la prostate, ou la glande thyroïde.

On définit ainsi un transducteur de traitement comme un transducteur adapté à émettre un faisceau d'ondes avec une intensité, une fréquence et une durée telles que les propriétés de la cible puissent être modifiées par l'application du faisceau. Par ailleurs, on définit un transducteur d'imagerie comme un transducteur adapté à émettre un faisceau d'ondes avec une intensité et une fréquence telles que les propriétés de la cible ne puissent pas être modifiées par l'application du faisceau.

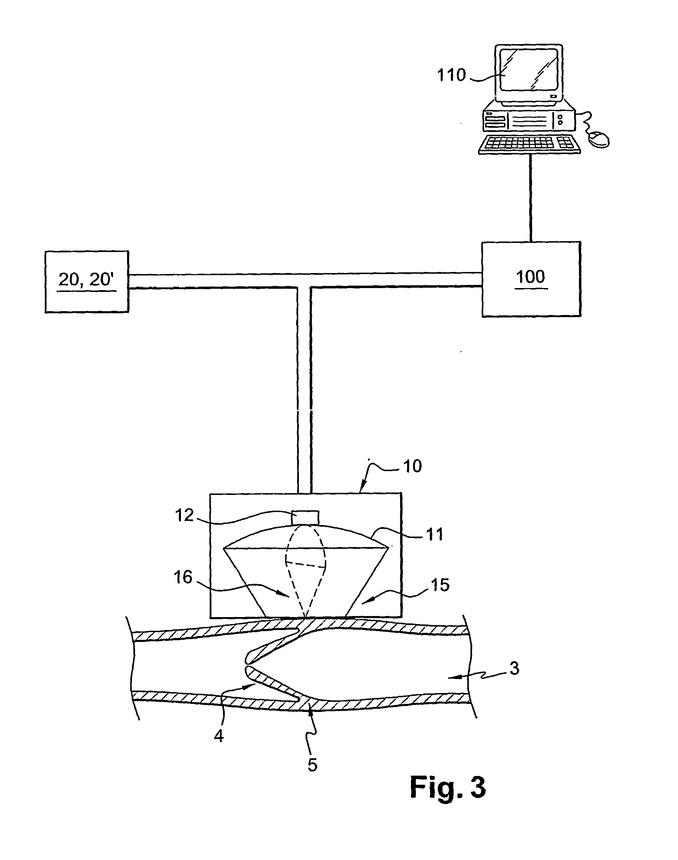

La figure 3 illustre schématiquement un dispositif de traitement par ultrasons selon l'invention.

Le dispositif selon l'invention comprend une sonde 10 intégrant au moins un transducteur de traitement 11 émettant un faisceau d'ondes ultrasonores présentant une surface d'onde asphérique. Le transducteur de traitement 11 est adapté à émettre un faisceau d'ondes ultrasonores de haute intensité focalisé avec une forte concentration de puissance, de l'ordre de 1KW/cm³. La sonde 10 peut en outre intégrer un transducteur d'imagerie 12, par exemple un transducteur d'échographe, connu en soi.

La sonde 10 est destinée à être placée en regard d'une zone à traiter. Dans l'exemple illustré, la zone à traiter est constituée par une veine 3 variqueuse. La sonde 10 peut être disposée contre la peau au niveau de l'anneau d'insertion 5 d'une valvule 4 pathologique. La sonde doit présenter un encombrement réduit afin que le clinicien puisse facilement repérer la zone cible à tout moment.

Selon l'invention, le transducteur de traitement 11 est adapté à focaliser les ondes ultrasonores dans le plan 15 de la veine selon une tache focale allongée dans la longueur de la veine 3. Le transducteur d'imagerie 12 peut être placé de manière à visualiser la veine dans un plan perpendiculaire 16 à celle-ci. Cette disposition n'est pas obligatoire bien que préférentielle car elle permet une meilleure vision par effet échographique de toute la largueur de la veine et de sa valvule.

La sonde du dispositif selon l'invention est reliée à au moins un générateur électronique. Un premier générateur d'onde 20 fournit et reçoit un courant haute fréquence au transducteur d'imagerie 12 pour une visualisation de la zone à traiter par échographie, éventuellement associées à un écho Doppler. Les signaux fournis au transducteur d'imagerie sont compris entre 5 et 20 MHz. Un générateur d'onde 20', distinct ou intégré au premier générateur 20, fournit des signaux de puissance au transducteur de traitement 11 pour une focalisation de forte intensité sur la zone à traiter. Les signaux fournis au transducteur de traitement sont compris entre 1 et 5 MHz.

La sonde du dispositif selon l'invention est également reliée à une unité de traitement électronique 100 adaptée à contrôler la sonde 10 et le générateur d'ondes 20. En particulier, l'unité de traitement 100 peut être adaptée à interpréter les signaux reçus par le transducteur d'imagerie 12 en mode échographie et/ou Doppler. L'unité de traitement 100 peut également être reliée à une interface homme-machine 110 pour une visualisation de la zone traitée et/ou pour une saisie de paramètres de fonctionnement.

Le dispositif selon l'invention est destiné à traiter une zone donnée par ultrasons focalisés de forte intensité (HIFU). Selon l'invention, le dispositif permet de focaliser les ultrasons sur une longueur donnée. En particulier dans le cas de traitement de veines variqueuses, il est particulièrement intéressant de provoquer une focalisation de forte intensité sur une longueur donnée de veine englobant le point d'attache de la valvule. On évite ainsi les problèmes de guidage de la sonde à ultrasons le long de la veine, ce qui est nécessaire dans le cas des dispositifs classiques utilisant une focalisation point par point.

Selon l'invention, la tache focale du transducteur de traitement 11, au lieu d'être ponctuelle comme dans la plupart des dispositifs connus, est allongée et inclinée par rapport à la direction de propagation des ultrasons, par exemple sur une longueur de l'ordre de 5 à 10 mm. De plus, contrairement à la ligne focale classiquement obtenue avec un transducteur cylindrique, la ligne de la tache focale 6 du dispositif selon l'invention est plus courte que la longueur du transducteur 11, garantissant ainsi une plus forte concentration de l'intensité acoustique. Par exemple, le transducteur pourra présenter un diamètre de l'ordre de 30 à 50 mm pour une tache focale de 5 à 10 mm.

Pour cela, le transducteur de traitement 11 du dispositif selon l'invention présente une surface asphérique. En particulier, toute surface quadratique peut être exploitée pour constituer tout ou partie de la surface d'émission du transducteur de traitement 11.

Selon la forme et la disposition du transducteur, la tache focale peut être perpendiculaire ou inclinée avec un angle plus ou moins important par rapport à la direction de propagation des ondes, par exemple avec un angle de 45° à 90°.

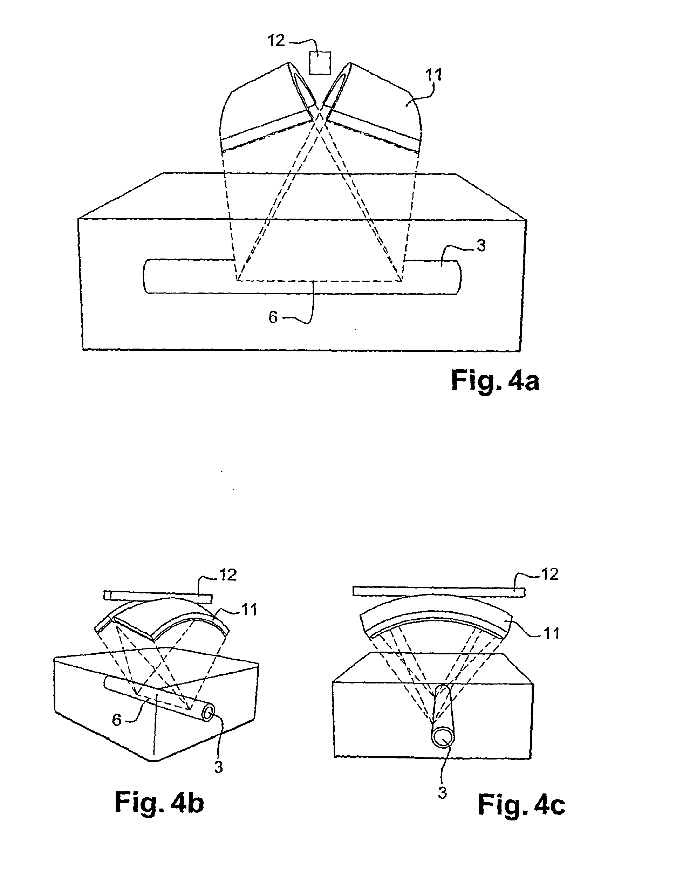

Selon un premier mode de réalisation, illustré sur les figures 4a à 4c, le transducteur de traitement 11 présente une surface d'émission conique. L'axe du cône formé par la surface d'onde du transducteur de traitement 11 est confondu avec la ligne de la tache focale 6. Selon le mode de réalisation illustré, deux transducteurs coniques sont disposés de manière à focaliser les ondes ultrasonores sur une même tache focale 6. La géométrie des surfaces d'émission des transducteurs de traitement et leur disposition relative sont telles que le croisement des faisceaux respectivement émis ne provoque pas d'annulation des ondes émises par lesdits transducteurs. La concentration acoustique est ainsi améliorée car les deux taches focales confondues 6 concentrent les ondes ultrasonores sur une ligne très fine et plus courte que la longueur de l'ensemble des deux transducteurs formant le transducteur de traitement 11.

La sonde intégrant le transducteur de traitement 11 est placée de sorte que la tache focale allongée 6 soit dans le plan de la veine 3 au niveau de la paroi présentant une varice. Ainsi, la portion déhiscente de la paroi veineuse peut être chauffée de façon à provoquer une rétractation localisée de collagène sur cette longueur de veine et refermer la valvule ce qui supprimera le reflux sanguin.

Le transducteur d'imagerie 12 peut être situé au centre du transducteur de traitement 11 et orienté de façon perpendiculaire au grand axe de la tache focale 6 afin d'observer toute la section de la veine 3. Plus spécifiquement, le transducteur d'imagerie 12 peut être placé dans l'espace libre entre les deux transducteurs de traitement 11.

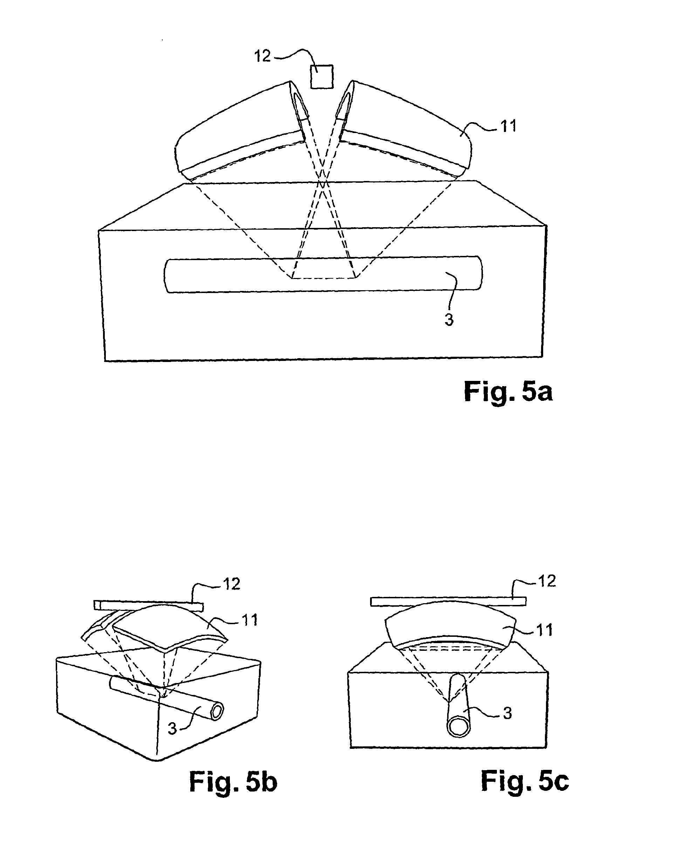

Selon un deuxième mode de réalisation, illustré sur les figures 5a à 5c, le transducteur de traitement 11 présente une surface d'émission ellipsoïdale. Selon le mode de réalisation illustré, deux transducteurs ellipsoïdaux sont disposés de manière à focaliser les ondes ultrasonores sur une même tache focale 6. Selon ce mode de réalisation, la géométrie des surfaces d'émission des transducteurs de traitement et leur disposition relative sont aussi telles que le croisement des faisceaux respectivement émis ne provoque pas d'annulation des ondes émises par lesdits transducteurs. La concentration acoustique est encore améliorée car les deux taches focales confondues 6 concentrant les ondes ultrasonores sur une ligne très fine et plus courte que chacun des transducteurs formant le transducteur de traitement 11.

Le transducteur d'imagerie 12 est situé au centre du transducteur de traitement 11, par exemple dans l'espace entre les deux transducteurs de traitement, et orienté de façon perpendiculaire au grand axe de la tache focale 6 afin d'observer toute la section de la veine 3.

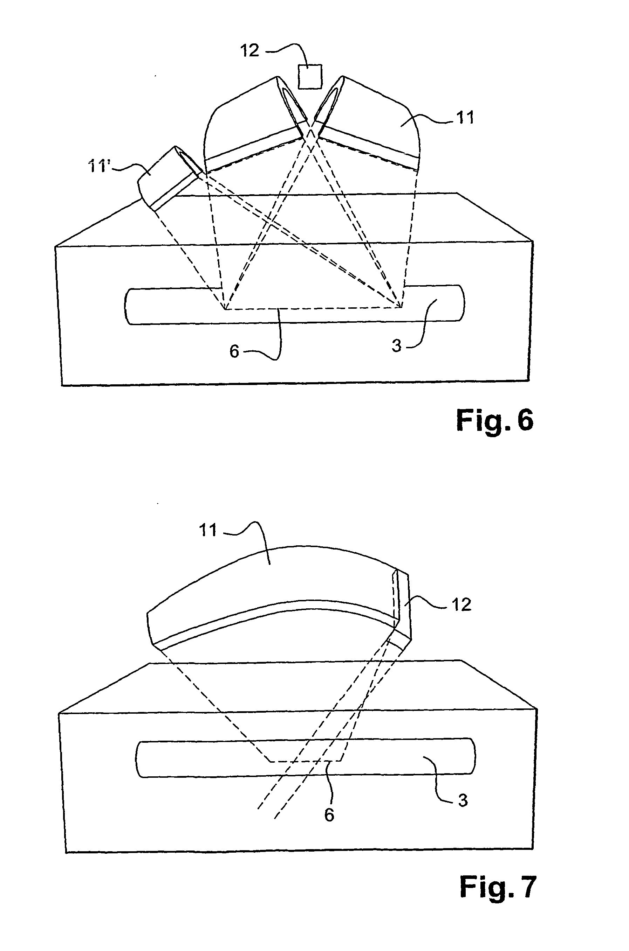

Selon un troisième mode de réalisation, illustré sur la figure 6, l'ensemble de transducteur de traitement comprend deux transducteurs 11 présentant une surface d'onde ellipsoïdale et un transducteur 11' présentant une surface d'onde conique, les transducteurs étant disposés de manière à focaliser les ondes ultrasonores sur une même tache focale 6. La disposition relative des transducteurs et leur géométrie sont telles que le croisement des faisceaux respectivement émis ne provoque pas d'annulation des ondes émises par lesdits transducteurs. La concentration acoustique est améliorée car les trois taches focales confondues 6 concentrent les ondes ultrasonores sur une ligne très fine et plus courte que l'ensemble des trois transducteurs formant le transducteur de traitement.

Le transducteur d'imagerie 12 peut être situé entre deux des transducteurs de traitement 11 et orienté de façon perpendiculaire au grand axe de la tache focale 6 afin d'observer toute la section de la veine 3.

Dans le mode de réalisation illustrée sur la figure 7, un unique transducteur de traitement 11 présentant une surface d'onde elliptique est associé à un transducteur d'imagerie 12 orienté de façon incliné par rapport au grand axe de la tache focale 6. Une telle orientation du transducteur d'imagerie permet de mesurer le flux sanguin dans la veine 3 en mode Doppler. La barrette du transducteur d'imagerie 12 est orientée de manière à inclure dans son plan de détection une partie de la ligne focale 6, et de préférence son centre. Bien entendu, une orientation inclinée du transducteur d'imagerie 12 peut être associée à toute forme asphérique, autre qu'elliptique, de la surface d'onde du transducteur de traitement 11 de l'appareil selon l'invention.

Le transducteur d'imagerie 12 peut être solidaire du transducteur de traitement 11. Selon un mode de réalisation, le transducteur d'imagerie 12 peut être accolé au transducteur de traitement 11, comme illustré sur la figure 7.

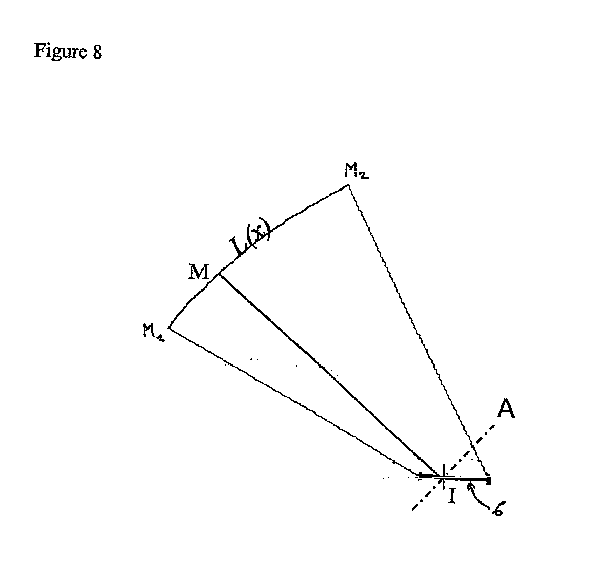

Selon un autre mode de réalisation, la surface d'onde asphérique peut être obtenue par construction de la manière suivante. La figure 8 illustre cette construction. Sur la figure 8, on a représenté une courbe L(x) servant de base à la construction de la surface d'onde du transducteur. Les ondes destinées à être émises par ce transducteur de traitement doivent se focaliser sur une tache 6 formant une ligne et désignée comme une ligne focale.

On choisit une courbe L(x) située dans le plan médian des transducteurs (plan de symétrie du dispositif), telle que toutes les normales à L(x) coupent la ligne focale. Pour chaque point M de la courbe L(x), l'intersection de la normale à la courbe avec la ligne focale est dénommée I. On développe ensuite L(x) sur l'espace ; on effectue pour chaque point M de la courbe L(x) une rotation autour de l'axe A défini comme la perpendiculaire à IM en I. On obtient ainsi à partir de chaque point M une portion de cercle dont l'ensemble forme la surface d'un des transducteurs. L'amplitude de la rotation définit la largeur des transducteurs et est limitée par le plan cutané. Lorsque deux transducteurs sont associés comme dans les exemples des figures 4a et 5a, il faut éviter que les ondes provenant du bord intérieur de l'un des transducteurs ne s'annulent avec les ondes provenant du bord extérieur de l'autre transducteur. Pour cela on choisira les points d'extrémités M₁, M₂ de la courbe L(x) de manière à ce que la différence entre les distances M₁I₁ et M₂I₂ soit un nombre entier de longueurs d'onde.

On obtient alors, pour chaque transducteur, une surface d'onde définie comme l'enveloppe d'une pluralité de cercles respectivement centrés sur une pluralité de points de la ligne focale et connectés entre eux de manière à ce que les deux normales en chaque points de l'enveloppe coupent la ligne focale.

Le dispositif selon l'invention peut être utilisé de la manière suivante.

Tout d'abord, la zone à traiter A est localisée. Le réseau veineux superficiel du patient est classiquement exploré par écho Doppler. Une telle localisation peut être effectuée directement avec le dispositif selon l'invention au moyen du transducteur d'imagerie intégré le cas échéant ou avec un autre dispositif de type échographique, éventuellement munie d'une fonction écho Doppler.

Le patient est généralement examiné en orthostatisme. La recherche d'un reflux peut faire appel à des manoeuvres de compression relaxation du mollet.

L'exploration par écho Doppler de la (des) veine(s) variqueuses recherche sur le trajet de cette (ces) veine(s) la (les) sites valvulaires pathologiques dont la localisation précise peut être matérialisée par un marquage cutané. Ces sites peuvent être caractérisés en écho Doppler couleur par l'existence d'un reflux partiel, commissural et/ou en échographie mode B par l'existence d'une dilatation de la paroi veineuse en regard de la commissure valvulaire qui écarte plus ou moins les deux feuillets de la valvule. C'est cette (ces) zone(s) dilatée(s) de la paroi veineuse que sera(ont) reconnue(s) comme zone(s) cible(s). Il s'agit principalement du bord externe de la paroi veineuse en regard des valvules sous ostiale et/ou huntérienne et/ou tronculaires pathologiques.

Le patient est alors généralement installé en décubitus.

La (les) zone(s) cible(s) de la veine à traiter est (sont) précisément identifiée(s) en regard des sites préalablement repérés lors de l'examen écho Doppler initial.

Au niveau de chaque site à traiter, la zone cible est matérialisée sur l'écran du système échographique. La focalisation du dispositif HIFU est ajustée de façon à correspondre avec la zone cible.

Par exemple, deux transducteurs de traitement sont disposés de façon symétrique par rapport à la zone à chauffer et sont suffisamment espacés pour placer une barrette de transducteur d'imagerie entre eux. Les faisceaux d'ondes émis par les deux transducteurs de traitement se concentrent sur une ligne d'environ 7 mm de long perpendiculaire au plan d'image du transducteur d'imagerie. La ligne de la tache focale est située dans le plan de la veine à une profondeur, sous la peau, de l'ordre de 15 mm. Le transducteur d'imagerie est placé au plus à 10 mm au dessus de la peau afin d'assurer une bonne qualité d'image sans gêner les faisceaux d'ondes émis par les transducteurs de traitement.

Avant chauffage, une anesthésie locale tumescente peut être pratiquée par infiltration d'une solution de Xylocaïne adrénalinée diluée tamponnée dans le fascia péri-saphénien. Outre son effet anesthésiant, cette infiltration va comprimer les parois veineuses, donnant une cible plus adaptée aux ultrasons.

Les ultrasons de puissance sont alors délivrés selon des abaques préétablis.

La paroi veineuse pathologiquement dilatée au niveau de l'anneau d'insertion des valvules est chauffée à une température adéquate, par exemple 75° à 100°C et de préférence 85 à 90°C, pendant 1 à 60 s, de préférence 10 à 20s. On veut obtenir ainsi la rétraction du collagène et in fine la rétraction de la paroi veineuse, sans détruire celle-ci ni les tissus environnants. Les puissances acoustiques utilisées sont de l'ordre de 20 à 35 W acoustiques. La fréquence ultrasonore est de l'ordre de 1 à 5 MHz, de préférence de 1,5 à 3 Mhz.

Ainsi, l'invention propose de traiter une pathologie variqueuse par échauffement de la paroi veineuse avec des ultrasons focalisés, sans coagulation ou occlusion de la veine. L'échauffement est de préférence conduit au niveau d'un anneau d'insertion d'une valvule pathologique.

L'effet de chaque séquence thérapeutique peut être immédiatement contrôlé par écho Doppler. On contrôle d'une part les modifications morphologiques de la paroi, tel que le calibre et l'épaisseur de la paroi, et d'autre part les modifications sur le plan hémodynamique, avec la persistance ou non d'un reflux. Le cas échéant la puissance, l'intensité ou le temps d'application des ultrasons peuvent être ajustés en fonction de ce contrôle. On peut également contrôler la paroi par élastographie.

Une ou plusieurs séquences complémentaires peuvent être réalisées jusqu'à l'obtention d'une restitution de la continence valvulaire.

Bien entendu, la présente invention n'est pas limitée aux modes de réalisation décrits à titre d'exemple; ainsi, bien que les transducteurs de traitement 11 aient été décrits avec des surfaces asphériques, ils sont équivalents à des ensembles transducteurs de traitements présentant des surfaces d'onde de forme sphérique associées à des lentilles cylindriques, ou à des ensembles transducteurs de traitements présentant des surfaces d'onde de forme plane associées à des lentilles elliptiques.

Dans ce qui précède, on a décrit des transducteurs dont la géométrie de la surface d'émission est identique à la forme de la surface d'onde voulue. Par exemple des transducteurs de forme conique émettront une surface d'onde conique lorsqu'ils sont reliés à un générateur électronique unique.

Cependant, les surfaces d'onde asphériques peuvent être obtenues à partir d'un transducteur de forme quelconque, par exemple sphérique ou plane, couplé à des moyens complémentaires. Selon une premier moyen, une lentille acoustique asphérique est interposée entre le transducteur et le tissu à traiter. Selon un second moyen, le transducteur est découpé en parties émissives indépendantes qui sont reliées chacune à un générateur de puissance. Ces générateurs fonctionnent suivant la même fréquence mais leurs phases sont décalées de façon à obtenir la surface d'onde désirée.

## Revendications

1. Dispositif à ultrasons comprenant un premier transducteur de traitement (11) adapté à émettre un faisceau d'ondes ultrasonores vers une zone à traiter (A), le faisceau présentant une surface d'onde asphérique et adapté à créer une tache focale (6) sur la zone à traiter, la tache focale (6) étant allongée et inclinée par rapport à la direction de propagation des ondes ultrasonores, **caractérisé en ce que** la longueur de la tache focale est inférieure à la longueur du transducteur, et **en ce que** la surface d'onde asphérique est autre que cylindrique.

2. Dispositif selon la revendication 1, **caractérisé en ce que** la tache focale (6) est perpendiculaire à la direction de propagation des ondes ultrasonores.

3. Dispositif selon la revendication 1 ou 2, **caractérisé en ce qu'**il comprend en outre un deuxième transducteur d'imagerie (12) adapté à visualiser la zone à traiter.

4. Dispositif selon la revendication 3, **caractérisé en ce que** le transducteur d'imagerie est adapté à visualiser un plan perpendiculaire à un grand axe de la tache focale (6).

5. Dispositif selon la revendication 3, **caractérisé en ce que** le transducteur d'imagerie est adapté à visualiser un plan incliné par rapport au grand axe de la tache focale (6).

6. Dispositif selon l'une des revendications 1 à 5, **caractérisé en ce que** le faisceau d'ondes émis par le transducteur de traitement (11) présente une surface d'onde de forme conique.

7. Dispositif selon l'une des revendications 1 à 5, **caractérisé en ce que** le faisceau d'ondes émis par le transducteur de traitement (11) présente une surface d'onde de forme ellipsoïdale.

8. Dispositif selon l'une des revendications 1 à 5, **caractérisé en ce que** le faisceau d'ondes émis par le transducteur de traitement (11) présente une surface d'onde de forme sphérique associée à une lentille cylindrique.

9. Dispositif selon l'une des revendications 1 à 5, **caractérisé en ce que** le faisceau d'ondes émis par le transducteur de traitement (11) présente une surface d'onde de forme plane associée à une lentille elliptique.

10. Dispositif selon l'une des revendications 1 à 5, **caractérisé en ce que** le transducteur de traitement (11) est découpé une pluralité de parties indépendantes adaptées à émettre chacune des faisceaux d'ondes ultrasonores à la même fréquence et déphasés entre eux de manière à former une surface d'onde asphérique.

11. Dispositif selon l'une des revendications 1 à 5, **caractérisé en ce que** le faisceau d'onde émis par le transducteur de traitement (11) présente une surface d'onde définie comme l'enveloppe d'une pluralité de cercles respectivement centrés sur une pluralité de points de la tache focale et connectés entre eux de manière à ce que les deux normales en chaque point de l'enveloppe coupent la tache focale.

12. Dispositif selon l'une des revendications 1 à 11, **caractérisé en ce qu'**il comprend une pluralité de transducteurs de traitement (11, 11') adaptés à focaliser des faisceaux d'ondes ultrasonores en une même tache focale (6) sur une zone à traiter.

13. Dispositif selon l'une des revendications 3 à 12, **caractérisé en ce que** le transducteur d'imagerie (12) est solidaire du transducteur de traitement (11).

14. Dispositif selon l'une des revendications 1 à 13, **caractérisé en ce qu'**il comprend en outre des moyens de contrôle (100) de la fréquence et/ou de la puissance des ondes ultrasonores émises par le premier transducteur de traitement (11) et/ou de la durée d'émission des ondes ultrasonores sur la surface à traiter.

15. Dispositif selon les revendications 3 et 14, **caractérisé en ce que** les moyens de contrôle (100) sont adaptés à interpréter des données fournies par le transducteur d'imagerie (12).

## Patentansprüche

1. Ultraschallvorrichtung, umfassend einen ersten Behandlungsschallkopf (11), der dazu geeignet ist, ein Ultraschallwellenbündel auf einen zu behandelnden Bereich (A) zu emittieren, wobei das Bündel eine asphärische Wellenfläche aufweist, und der dazu geeignet ist, um einen Fokusfleck (6) auf dem zu behandelnden Bereich zu erstellen, wobei der Fokusfleck (6) länglich und im Verhältnis zur Ausbreitungsrichtung der Ultraschallwellen geneigt ist, **dadurch gekennzeichnet, dass** die Länge des Fokusflecks kleiner als die Länge des Schallkopfes ist, und dass die asphärische Wellenfläche anders als zylindrisch ist.

2. Vorrichtung nach Anspruch 1, **dadurch gekennzeichnet, dass** der Fokusfleck (6) zur Ausbreitungsrichtung der Ultraschallwellen rechtwinklig ist.

3. Vorrichtung nach Anspruch 1 oder 2, **dadurch gekennzeichnet, dass** sie ferner einen zweiten Bildgebungsschallkopf (12) umfasst, der dazu geeignet ist, um den zu behandelnden Bereich zu visualisieren.

4. Vorrichtung nach Anspruch 3, **dadurch gekennzeichnet, dass** der Bildgebungsschallkopf dazu geeignet ist, eine Ebene zu visualisieren, die zu einer langen Achse des Fokusflecks (6) rechtwinklig ist.

5. Vorrichtung nach Anspruch 3, **dadurch gekennzeichnet, dass** der Bildgebungsschallkopf dazu geeignet ist, um eine Ebene zu visualisieren, die im Verhältnis zu der langen Achse des Fokusflecks (6) geneigt ist.

6. Vorrichtung nach einem der Ansprüche 1 bis 5, **dadurch gekennzeichnet, dass** das Wellenbündel, das von dem Behandlungsschallkopf (11) emittiert wird, eine konische Wellenfläche aufweist.

7. Vorrichtung nach Anspruch 1 bis 5, **dadurch gekennzeichnet, dass** das Wellenbündel, das von dem Behandlungsschallkopf (11) emittiert wird, eine elliptische Wellenfläche aufweist.

8. Vorrichtung nach einem der Ansprüche 1 bis 5, **dadurch gekennzeichnet, dass** das Wellenbündel, das von dem Behandlungsschallkopf (11) emittiert wird, eine sphärische Wellenfläche aufweist, die mit einer zylindrischen Linse verbunden ist.

9. Vorrichtung nach einem der Ansprüche 1 bis 5, **dadurch gekennzeichnet, dass** das Wellenbündel, das von dem Behandlungsschallkopf (11) emittiert wird, eine ebene Wellenfläche aufweist, die mit einer elliptischen Linse verbunden ist.

10. Vorrichtung nach einem der Ansprüche 1 bis 5, **dadurch gekennzeichnet, dass** der Behandlungsschallkopf (11) in eine Vielzahl von unabhängigen Teilen unterteilt ist, die dazu geeignet sind, um jeweils Ultraschallwellenbündel auf der gleichen Frequenz und untereinander phasenverschoben zu emittieren, um eine asphärische Wellenfläche zu bilden.

11. Vorrichtung nach einem der Ansprüche 1 bis 5, **dadurch gekennzeichnet, dass** das Wellenbündel, das von dem Behandlungsschallkopf (11) emittiert wird, eine Wellenfläche aufweist, die als Hüllkurve einer Vielzahl von Kreisen definiert ist, die jeweils um eine Vielzahl von Punkten des Fokusflecks zentriert sind und miteinander verbunden sind, so dass die beiden Senkrechten in jedem Punkt der Hüllkurve den Fokusfleck schneiden.

12. Vorrichtung nach einem der Ansprüche 1 bis 11, **dadurch gekennzeichnet, dass** sie eine Vielzahl von Behandlungsschallköpfen (11, 11') umfasst, die dazu geeignet sind, um Ultraschallwellenbündel in ein und demselben Fokusfleck (6) auf einem zu behandelnden Bereich zu fokussieren.

13. Vorrichtung nach einem der Ansprüche 3 bis 12, **dadurch gekennzeichnet, dass** der Bildgebungsschallkopf (12) mit dem Behandlungsschallkopf (11) fest verbunden ist.

14. Vorrichtung nach einem der Ansprüche 1 bis 13, **dadurch gekennzeichnet, dass** er ferner Mittel (100) zum Regeln der Frequenz und/oder der Leistung der Ultraschallwellen, die von dem ersten Behandlungsschallkopf (11) emittiert werden, und/oder der Emissionsdauer der Ultraschallwellen auf der zu behandelnden Oberfläche umfasst.

15. Vorrichtung nach Anspruch 3 und 14, **dadurch gekennzeichnet, dass** die Regelmittel (100) dazu geeignet sind, um Daten auszulegen, die von dem Bildgebungsschallkopf (12) bereitgestellt werden.

## Claims

1. An ultrasound device comprising a first treatment transducer (11) which can transmit a beam of ultrasound waves to an area to be treated (A), the beam having an aspherical wavefront, and which is adapted to create a focal spot (6) on the area to be treated, the focal spot (6) being elongated and inclined with respect to the direction of propagation of the ultrasound waves, **characterised in that** the length of the focal spot is less than the length of the transducer, and **in that** the aspherical wavefront is other than cylindrical.

2. The device according to claim 1, **characterized in that** the focal spot (6) is perpendicular to the direction of propagation of the ultrasound waves.

3. The device according to claim 1 or 2, **characterized in that** it also comprises a second imaging transducer (12) which is able to display the area to be treated.

4. The device according to claim 3, **characterized in that** the imaging transducer is able to display a plane perpendicular to a major axis of the focal spot (6).

5. The device according to claim 3, **characterized in that** the imaging transducer is able to display a plane inclined with respect to the major axis of the focal spot (6).

6. The device according to one of claims 1 to 5, **characterized in that** the beam of waves transmitted by the treatment transducer (11) has a wavefront with a conical shape.

7. The device according to one of claims 1 to 5, **characterized in that** the beam of waves transmitted by the treatment transducer (11) has a wavefront with an ellipsoid shape.

8. The device according to one of claims 1 to 5, **characterized in that** the beam of waves transmitted by the treatment transducer (11) has a wavefront with a spherical shape associated with a cylindrical lens.

9. The device according to one of claims 1 to 5, **characterized in that** the beam of waves transmitted by the treatment transducer (11) has a wavefront with a flat shape associated with an elliptical lens.

10. The device according to one of claims 1 to 5, **characterized in that** the treatment transducer (11) is separated into a plurality of independent parts which are each able to transmit beams of ultrasound waves at the same frequency and phase shifted amongst them so as to form an aspherical wavefront.

11. The device according to one of claims 1 to 5, **characterized in that** the wave beam transmitted by the treatment transducer (11) has a wavefront defined as the envelope of a plurality of circles respectively centred on a plurality of points of the focal spot and connected to each other in such a way that the two normals at each point of the envelope intersect the focal spot.

12. The device according to one of claims 1 to 11, **characterized in that** it comprises a plurality of treatment transducers (11, 11') which are able to focus beams of ultrasound waves onto the same focal spot (6) on an area to be treated.

13. The device according to one of claims 3 to 12, **characterized in that** the imaging transducer (12) is integral with the treatment transducer (11).

14. The device according to one of claims 1 to 13, **characterized in that** it also comprises control means (100) for the frequency and/or the power of the ultrasound waves transmitted by the first treatment transducer (11) and/or the duration of transmission of the ultrasound waves onto the surface to be treated.

15. The device according to claims 3 and 14, **characterized in that** the control means (100) are able to interpret data provided by the imaging transducer (12).
